# EUROPEAN PATENT SPECIFICATION

(11) **EP 4 132 354 B1**
(45) Date of publication and mention of the grant of the patent: **01.05.2024**
(21) Application number: 21727211.1
(22) Date of filing: 08.04.2021
(51) Int. Cl.: A61B 5/03, A61B 5/00

(54) **IMPLANTABLE BILIARY OR PANCREATIC STENT AND MANUFACTURE METHOD THEREOF**
IMPLANTIERBARER GALLEN- ODER PANKREASSTENT UND HERSTELLUNGSVERFAHREN DAFÜR
ENDOPROTHÈSE BILIAIRE OU PANCRÉATIQUE IMPLANTABLE ET SON PROCÉDÉ DE FABRICATION

(30) Priority: 08.04.2020 PT 2020116248
(43) Date of publication of application: 15.02.2023
(73) Proprietor: Hydrumedical, S.A., 4805-017 Barco (PT)
(72) Inventor: ANTUNES BARROS, Alexandre António, 4805-156 Caldas da Taipas (PT); PIRES SEPÚLVEDA, Catarina, 4710-407 Braga (PT); RODRIGUES DE LIMA, Estevão Augusto, 4050-499 Porto (PT); BRAGANÇA, Pedro, 4810-025 Guimarães (PT); GONÇALVES DOS REIS, Rui Luís, 4250-242 Porto (PT); CARVALHEIRA NEVES, Sara, 4715-225 Braga (PT); DA CUNHA FERNANDES, Helena Filipa, 4760-034 Vila Nova de Famalicão (PT); SIMÕES COSTA, Hugo Filipe, 4760-034 Vila Nova de Famalicão (PT); FERNANDES DA SILVA ANDRADE LEITE, André Filipe, 4760-034 Vila Nova de Famalicão (PT); DA CRUZ PEIXOTO, Pedro Tiago, 4760-034 Vila Nova de Famalicão (PT); GONÇALVES DE MATOS, Bruno Guilherme, 4760-034 Vila Nova de Famalicão (PT); DA SILVA SOARES, Juliana Patrícia, 4760-034 Vila Nova de Famalicão (PT)
(74) Representative: Patentree
(86) International application number: PCT/IB2021/052926
(87) International publication number: WO 2021/205379

(56) References cited:
- FR-A1- 3 046 676
- US-A1- 2009 131 767
- US-A1- 2019 133 472
- LEUNG ET AL: "Biliary stents", TECHNIQUES IN GASTROINTESTINAL ENDOSCOPY, W.B. SAUNDERS, AMSTERDAM, NL, vol. 4, no. 3, 1 July 2002 (2002-07-01), pages 164-171, XP005468425, ISSN: 1096-2883, DOI: 10.1053/TGIE.2002.33793

## Description

### Technical field

The present disclosure relates to the field of implantable medical devices, more in particularly, the disclosure relates to a functionalized implantable biliary or pancreatic stent.

### Background

Alterations on the physiological parameters on the different regions of the gastrointestinal tract are often indicative of medical problems. The internal pressure is one of the essential parameters to be assessed in order to prevent and/or solve complications.

Several medical conditions can alter the gastrointestinal physiology, thus interfering with its normal function. These include stenosis caused by cancer of the stomach, duodenum, gallbladder or pancreas; gastrointestinal compression caused by malignant lymphadenopathy or widespread peritoneal deposits; biliary fistulae after biliary surgery or bile duct stones. These physiopathological alterations can translate into gastrointestinal disfunction and asymptomatic alterations.

The abovementioned conditions can be ameliorated by expanding the obstructed area. Stents are the most common method to clear the narrowed ducts. Regardless, the stent might itself become obstructed along time, so that the obstruction may recur, together with several complications associated to the obstruction.

To avoid severe complications, patients need to regularly visit the hospital, to check the stent status. However, if complications occur in between the monitoring appointments, the obstruction will not be timely detected potentially leading to the development of serious complications. As example, cholangitis, jaundice or sepsis may occur due to biliary stent occlusion.

Therefore, there is a need for the development of tools that allow a non-invasive assess to the real-time status of the stents and the ducts where they are inserted, particularly to monitor the degree of obstruction of the ducts.

As described on the state of the art, one way to achieve such monitoring is the continuous measurement of the duct pressure.

Document US20190133472 relates to a system for monitoring a pressure in a biliary tract includes: a stent for a biliary tract including a pressure sensor; and a subcutaneous implant medical device including a communication module which receives a measured value of a pressure in a biliary tract through communication with the pressure sensor and a power module which supplies a power to the pressure sensor.

Document CN106943137 A discloses a biliary tract pressure detecting device, comprising a pipe with a sensor head. The device is designed to be used on pinpointed assessment of biliary pressure, in a medical appointment context.

Document CN106901714 A discloses a piezometric tube for measuring gallbladder pressure, that requires a wired connection between the tube and an external receiving equipment.

Document US20070027495A1 relates to o a sensor that is implantable to sense bladder condition. The disclosure describes an implantable bladder sensor that is attachable to an exterior surface of a urinary bladder to sense bladder condition or activity.

Document US2009131767 A relates to implantable devices for evaluating body-associated fluid transport structures. The implantable devices include vascular, urinary, biliary, or gastrointestinal tract stents. The implantable devices may include two or more pressure sensors spaced a distance apart from each other on the fluid transport structure. With this configuration, pressure measurements may be obtained from the sensors and any differences in the observed measurements determined. The sensors and a processor configured to process data may be positioned at one end of the elongated structure of the device.

Besides the clear clinical advantages, the development of less invasive methods, compatible with continuous and long-term monitoring in a more natural context, may allow more accurate studies regarding the implications that pressure variations within the gastrointestinal tract may have on the different structures involved.

These facts are disclosed in order to illustrate the technical problem addressed by the present disclosure.

### Summary of the Invention

The present invention relates to an implantable biliary or pancreatic stent as defined in claim 1, a kit comprising an implantable biliary or pancreatic stent as defined in claim 14 and a manufacture method of an implantable biliary or pancreatic stent as defined in claim 15. Preferred embodiments of the invention are defined in the dependent claims.

### General Description

An aspect of the present disclosure relates to an implantable biliary or pancreatic stent comprising a first end for placing in the bile duct or the pancreatic duct and a second end, each said end comprising a pressure sensor arranged to measure bile duct or pancreatic duct pressure, respectively.

In an embodiment, the sensors are configured to detect a differential pressure between the bile duct or the pancreatic duct, and duodenum.

Advantageously, the two sensors are electrically independent, that is, they are not connected electrically. This has benefits in the data acquisition process, reducing possible interferences and ensures that sensor's readings are totally independent from each other. This also simplifies the stent construction, especially taking into account the distance between the two sensors.

An aspect of the present disclosure relates to an implantable biliary or pancreatic stent for implanting in the gastrointestinal tract, comprising a first end for placing in the bile duct or the pancreatic duct and a second end for placing in the duodenum, the first end comprising a pressure sensor arranged to measure bile duct or pancreatic duct pressure, respectively, and the second end comprising a pressure sensor arranged to measure duodenal pressure.

The presence of a biliary or pancreatic stent inside the gastrointestinal tract influences the pressure noticed along the ducts, in particular the bile duct or the pancreatic duct. The measurement of the pressure at the bile duct or pancreatic duct, and duodenum should be as exact as possible, to correctly evaluate the condition of the stent and the gastrointestinal tract where the stent is inserted. For safeguarding the gastrointestinal tract condition (including encrustations, obstructions, incorrect placement of the stent, appropriate flow of bile along the bile duct, appropriate flow of pancreatic juices along the pancreatic duct, in particular within the stent and/or around the stent), it is fundamental to evaluate not only the pressure at the bile duct or pancreatic duct, and duodenal pressures, but also the differential pressure between the bile duct or the pancreatic duct, and the duodenum. Considering a long-term application, the stent should be a continuous tube that connects the bile duct, or pancreatic duct, with the duodenum, promoting the drainage along the ducts, in particular within the stent and/or around the stent. If the drainage is compromised, complications may occur at gallbladder and pancreas level, leading to the development of serious complications, such as cholangitis, jaundice or sepsis.

In an embodiment, each pressure sensor comprises an electronic circuit with electronic components and a substrate for receiving the electronic circuit and electronic components, wherein said substrate is a flexible membrane.

In an embodiment, the flexible membrane is a sleeve surrounding the stent or the flexible membrane is a flexible tube that is part of a thin tube that forms the stent, in particular the flexible membrane having a thickness of 80-150 µm.

The electronic components of the implantable biliary or pancreatic stent, as described in previous embodiments, are connected by wire-bonding.

In an embodiment, each pressure sensor comprises a flexible printed circuit board (PCB) having soldered electronic components.

In an embodiment, one or more of said sensors comprises an antenna for receiving power wirelessly.

In an embodiment, one or more of said sensors comprises an antenna for transmitting data wirelessly.

In an embodiment, the antenna is a near-field communication, NFC, antenna.

In an embodiment, the implantable biliary or pancreatic stent of the present disclosure may comprise a liquid-tight encapsulation of said pressure sensors.

In an embodiment, the implantable biliary or pancreatic stent of the present disclosure may have a diameter inferior to 3 mm, preferably 2.5-2 mm.

In an embodiment, the pressure sensor placed on the first end is configured to detect a relative pressure of 0 up to 200 cm H₂O (19.6 kPa).

In an embodiment, the pressure sensor placed on the second end is configured to detect a relative pressure of 0 up to 100 cmH₂O (9.8 kPa).

In an embodiment, the sensors are configured to detect a differential pressure between the first end and the second end of up to 200 cmH₂O (19.6 kPa).

In an embodiment, the transmitter comprises an antenna that comprises an operation frequency of 6-60 MHz, in particular 13.56 MHz.

In an embodiment, each sensor has an elongated antenna arranged longitudinally along the stent.

In an embodiment, each sensor comprises two antennas placed diametrically opposite in respect of the stent. In particular, a first antenna for receiving power wirelessly and a second antenna for transmitting data wirelessly, preferably both antennas being NFC-frequency antennas.

In an embodiment, the plurality of pressure sensors is selected from capacitive sensor, piezoresistive sensor, or combinations thereof.

In an embodiment, the implantable stent of the present disclosure may further comprise a pH sensor, a temperature sensor, a flow sensor, a volume sensor, or combinations thereof.

In an embodiment, the implantable stent of the present disclosure may further comprise an electronic data processor arranged to detect and calculate local pressure. In an embodiment, the electronic data processor is arranged to detect and calculate pressure during bile discharge or pancreatic juice secretion.

In an embodiment, the electronic data processor is arranged to calculate the differential pressure between two separate locals, defined by the placement of the first end and the second end. In an embodiment, the first end is placed in the bile duct or the pancreatic duct, and a second end is placed in the duodenum.

The sensors may be applied to the external surface of the stent, thus allowing the measurement of the environmental pressure on the vicinities of the pressure sensors.

It is also described a kit comprising the implantable biliary or pancreatic stent according to any of the disclosed embodiments and an external reader comprising an antenna and an electronic circuit for communicating wirelessly with said stent.

Another aspect of the present disclosure relates to a manufacture method for providing an implantable biliary or pancreatic stent of the present disclosure, comprising the steps of:
providing an implantable biliary or pancreatic stent for implanting in the gastrointestinal tract, comprising a first end for placing in the bile duct or the pancreatic duct and a second end for placing in the duodenum;
providing the first end with a pressure sensor arranged to measure bile duct or pancreatic duct pressure, respectively, and providing the second end with a pressure sensor arranged to measure duodenal pressure.

### Brief Description of the Drawings

The following figures provide preferred embodiments for illustrating the description and should not be seen as limiting the scope of invention.

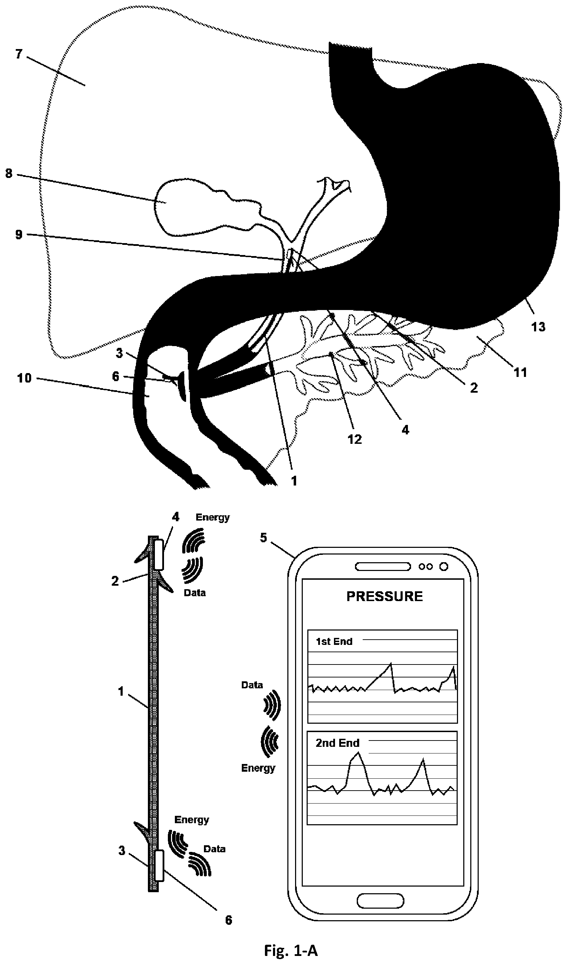

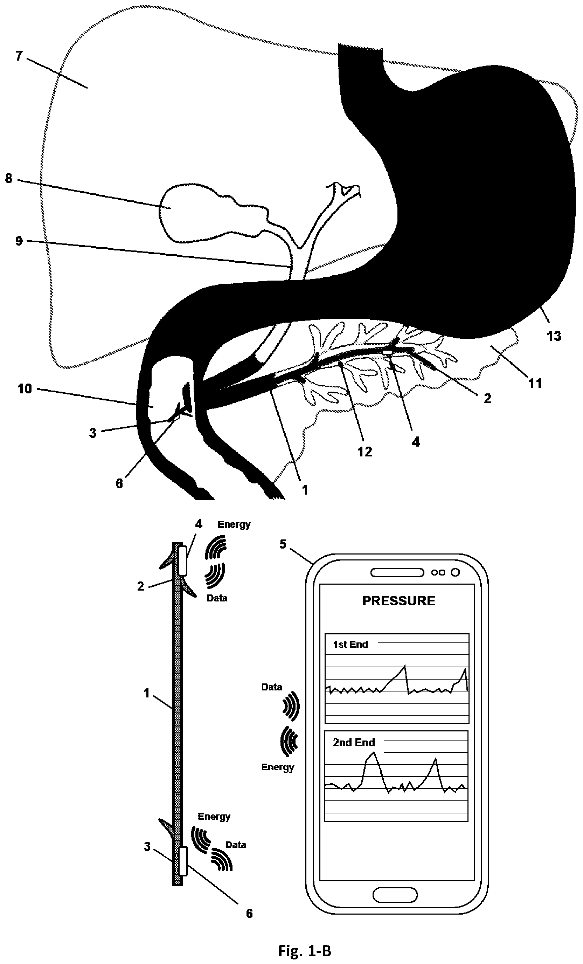

**Figure 1** shows a schematic representation of embodiments of a wireless implantable biliary stent (Figure 1-A) and a wireless pancreatic stent (Figure 1-B). The representation shows an implantable stent 1 integrating a wireless electronic component **4,6** that may comprise a sensor, an interface and a transmitter to monitor the local pressure. The system is placed internally in the gastrointestinal tract. The biliary stent (Figure 1-A) is placed in the bile duct **9** (that connects the liver **7** and the gallbladder **8** to the duodenum **10**), wherein the first end **2** is placed into the bile duct **9** and the second end **3** is located at the duodenum **10.** The pancreatic stent (Figure 1-B) is inserted in the pancreatic duct **12** (that connects the pancreas **11** and the duodenum **10**), wherein the first end **2** is inserted into the pancreatic duct and the second end **3** is placed at the duodenum **10.** Stomach is represented as **13.** The electronic component **4**, **6** may contain a transmitter receiving energy from a mobile wireless device **5** and transmitting it to the remaining components.
**Figure 2** shows a schematic representation of an embodiment of the interface system of the implantable stent of the present disclosure that establishes the communication between the transmitter and the sensor. The sensor may receive the energy and then captures and transmits the pressure data acquired back to the interface. On its turn, the interface passes the sensor information to the transmitter. The transmitter emits the collected data via the antenna to a wireless device 5. The wireless device 5 functions both as energy emitter and data receiver/analyzer.
**Figure 3** shows a schematic representation of an embodiment of the interface system of the implantable stent of the present disclosure that establishes the communication between NFC transmitter and sensor.
**Figure 4** depicts a schematic drawing of an embodiment of the device to be integrated on the stent comprising a pressure sensor **14**, an analog-to-digital converter **15**, a microcontroller unit **16**, an wireless transceiver **17** and an wireless antenna **18**, in particular the wireless communications being NFC.
**Figure 5** depicts an embodiment of the arrangement of the NFC antennas **18** on the stent **1**.
**Figure 6** is a schematic representation of an embodiment of the external reader, comprising an NFC initiator/HF Reader **19**, a microcontroller unit **20**, Bluetooth **21**, USB connector **22**, rechargeable battery **23**, charge management circuit **24**, and an NFC reader antenna **25**.
**Figure 7** is a schematic representation of an embodiment of the device coupled to a biliary or pancreatic stent **1**, showing the PCB electronic system **26** and the stent antenna **18**.

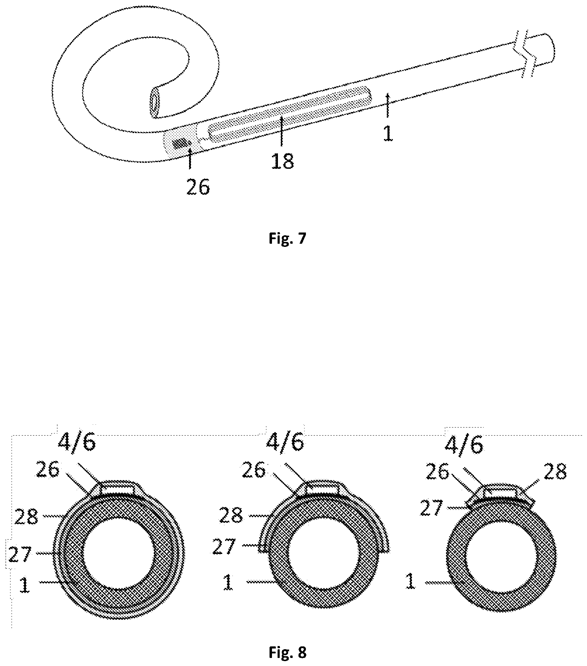

**Figure 8** is a schematic representation of the stents' cross-section in the electronic component region, showing the placement of the electronic component **4** or **6**, and the PCB **26** on the stent **1**. An internal coating/sleeve **27** is placed between the stent **1** and the PCB **26**. This internal coating/sleeve may cover all the stent or only part of it. The electronic component **4**, **6**, and the PCB **26** are covered by an external coating/sleeve **28**, that can be designed to cover all the stent, or only a part of it, providing that the electronic component and the PCB are covered by it.

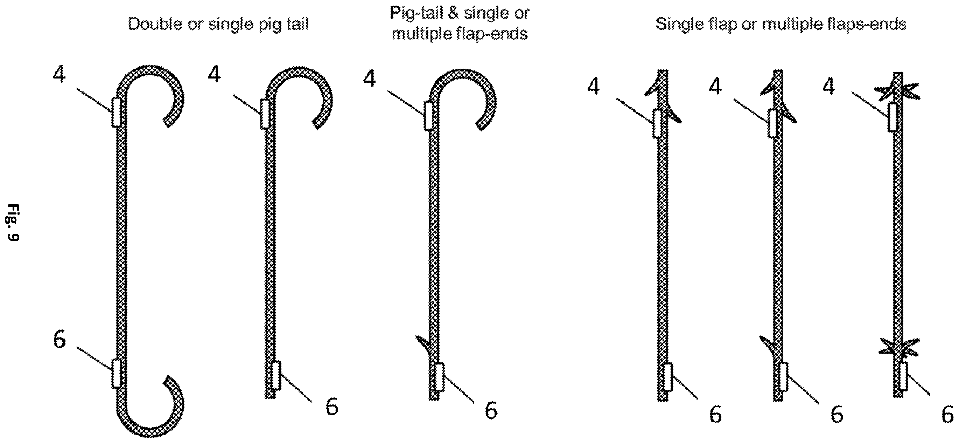

**Figure 9** schematically shows different embodiments of biliary and pancreatic stents and the placement of the electronic components **4, 6**, whether for polymeric or metallic embodiments.

### Detailed Description

The present invention relates to an implantable biliary or pancreatic stent comprising a first end **2** for placing in the bile duct **9** or the pancreatic duct **12,** and a second end **3** for placing in the duodenum, the first end comprising a pressure sensor **4** arranged to measure bile duct or pancreatic duct pressure, respectively, and the second end comprising a pressure sensor **6** arranged to measure duodenal pressure. Each pressure sensor comprises an electronic circuit with electronic components and a substrate for receiving the electronic circuit and electronic components, wherein said substrate is a flexible membrane. The flexible membrane is a sleeve surrounding the stent or the flexible membrane is a flexible tube that is part of a thin tube that forms the stent, in particular the flexible membrane may have a thickness of 80-150 µm. The electronic components may be connected by wire-bonding. Each pressure sensor may comprise a flexible PCB (26) having soldered electronic components. A manufacture method for providing said implantable biliary or pancreatic stent is also disclosed.

In an embodiment, the implantable biliary or pancreatic stent of the present disclosure is a portable homecare monitoring solution for measuring pressure within the gastrointestinal tract, preferably within the pancreatic and/or biliary duct.

In an embodiment, the sensor's data is collected externally, transmitted via the NFC antenna, and monitored in a portable device (smartphone, tablet, etc.) using a dedicated application.

In an embodiment, the implantable biliary or pancreatic stent of the present disclosure comprises at least two pressure sensors, an interface for example a microcontroller, and a transmitter.

In an embodiment, the sensors are configured to detect a differential pressure between the bile duct or the pancreatic duct, and the duodenum.

In an embodiment, the implantable biliary or pancreatic stent of the present disclosure comprises a biliary or pancreatic stent integrated with wireless pressure sensors and near-field communication (NFC) antenna. Alternatively, other wireless short-range communications may be used, for example a body area network communication, or a Bluetooth communication, or a communication in UHF, ultra-high frequency.

In an embodiment, the pressure sensor is selected from a list of capacitive sensors, piezoresistive sensor, or combinations thereof.

In an embodiment, the pressure sensor is wireless (based on microelectromechanical system, MEMS) and the antenna is a near-field communication (NFC) antenna.

In an embodiment, the system is battery-free, and therefore the system harvests energy provided by and external power source.

In an embodiment, a power emitting source provides energy wirelessly via the NFC antenna.

In an embodiment, the MEMS system is then able to measure the pressure values and sends back this information.

In an embodiment, the external power source may be a portable device such as a mobile phone or a computer, in particular that also collects and analyses the data.

In an embodiment, the electronic components are assembled to the stent using an adhesive/glue. The assembly is then coated/encapsulated with a biocompatible coating.

In an embodiment, the implantable biliary or pancreatic stent of the present disclosure may further comprise a pH sensor, temperature sensor, flow sensor, volume sensor, or combinations thereof, among others.

In an embodiment, the implantable biliary or pancreatic stent of the present disclosure can be divided in two main components: the biliary or pancreatic stent, and the electronic component.

In an embodiment, the implantable biliary or pancreatic stent of the present disclosure may comprise silicone, polyurethane, or mixtures thereof, among others.

In an embodiment, the electronic component may comprise the different subcomponents, that are encapsulated and isolated from the surrounding environment using a biocompatible resin.

In an embodiment, the implantable biliary or pancreatic stent of the present disclosure comprises a pressure sensor that acquires and emits the recorded data wirelessly.

The disclosure includes the following advantages:
the disclosure has a pressure sensor that acquires and emits the recorded data wirelessly; some current methods use pressure sensors connected (via wires) to external devices;
some current solutions do not simulate the patient's daily routine and do not allow the patient to go home and monitor the intra-body pressures;
some current solutions have an associated physiological conditioning (it is performed in the artificial environment of a clinical setting).

In an embodiment, the diameter of the sensor-stent assembly should be of 3 mm or less to fit in small gastrointestinal ducts, such as the biliary or pancreatic ducts. Also, the sensor-stent assembly does not have an energy storage unit, being then necessary to select low energy consumption components.

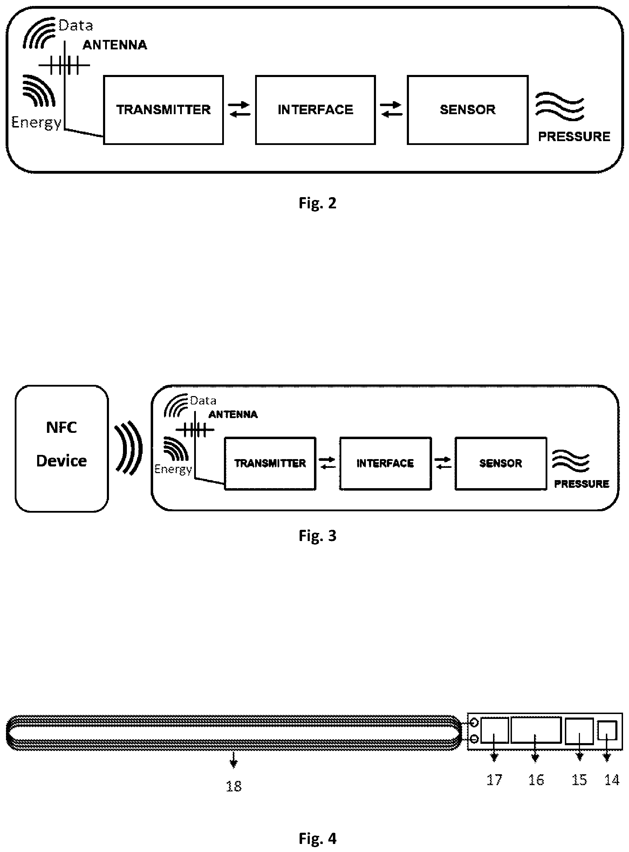

Figure 4 shows an embodiment of the device to be integrated on the stent.

The present disclosure is more particularly described in the following example that is intended as illustrative only since numerous modifications and variations therein will be apparent to those skilled in the art.

In an embodiment, it was selected the Murata's capacitive absolute pressure sensor SCB10H **14.** This family of sensors has a wide range of pressures, being B012 the most interesting series, since it works for a range between 0 and 1220 cmH₂O. In addition, it is a capacitive sensor based on MEMS that is not encapsulated, allowing a customized coupling for the application intended in this disclosure. The high resistance and low passive capacitance insulation of this sensor allows for very low energy consumption, high stability and accuracy over time and temperature variations. That is advantageous since measurements cannot be affected by external factors such as humidity, temperature and mechanical or chemical shocks and it will have to last for at least the lifetime of the implanted stent. Additionally, this sensor will be encapsulated with a thin biocompatible silicone.

The analog-to-digital signal converter **15**, in addition to being specific to the sensor that has been selected, it meets certain requirements, such as dimensions, power consumption, operating range and interface. In an embodiment, it was used a Renesas' ZSSC3123, a CMOS integrated circuit for precise conversion of capacitances into digital signal and specific signal correction from capacitive sensors. The digital compensation of the sensor offset, sensitivity and temperature deviation are performed by means of an internal digital signal processor executing a correction algorithm with calibration coefficients stored in a non-volatile EEPROM. The data acquired and corrected by this component are sent to the microcontroller (master) **16**, that manages the communication through a Serial Peripheral Interface (SPI) protocol. The microcontroller is also responsible for controlling communication with the wireless transmitter by an I2C digital interface.

Whenever the system receives energy, it starts to acquire data from the pressure sensor, which is processed and filtered on a first stage through the interface between the sensor and the transmitter. The microcontroller is also responsible for controlling the communication with the wireless transmitter. In an embodiment, a STM8 microcontroller from STMicroelectronics was selected, essentially due to its dimensions, low consumption and compatible communication interface with the transmitter (I2C).

Connected to the microcontroller is a radio-frequency identification (RFID) transmitter, the integrated circuit (IC) responsible for sending data to an exterior receiver device. Communication between the stent and the exterior reader is established through NFC technology, at a frequency of 13.56MHz. Due to the frequency of operation and communication protocol used, it is necessary that this controller supports the specifications established for NFC communication. In an embodiment, a STMicroelectronics controller ST25DV04K was selected as NFC transceiver **17**. Another main functionality of this component is the possibility to harvest electromagnetic energy through the antenna attached to it. In this way, it can not only supply its own internal circuit, but also supply power to external components, such as the microcontroller and the sensor. It is therefore, an indispensable component of this non-invasive wireless communication sensor system. This is physically connected to the microcontroller through a digital interface (I2C) and endows the wireless communication system with the external reader through the physical connection of an antenna.

In an embodiment, the loop antenna **18** is based on a 0.1 mm copper wire coil, fabricated on a flexible substrate. It has a length and width of 6 cm and 0.25 cm, respectively, with an inductance less than or equal to 4.83 µH. It is connected to the terminals of the ST25DV04K. The NFC antenna-controller is compensated with an external synchronization capacitor in the pF range to improve the system's range.

In an embodiment, each sensor has an elongated antenna arranged longitudinally along the stent.

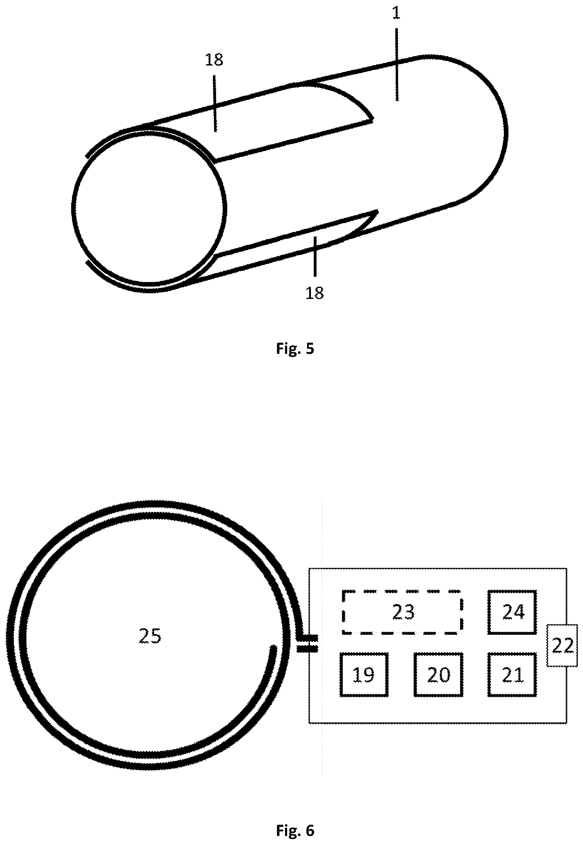

In an embodiment, each sensor comprises two antennas placed diametrically opposite in respect of the stent (Figure 5). In particular, a first antenna for receiving power wirelessly and a second antenna for transmitting data wirelessly, preferably both antennas being NFC-frequency antennas.

This solution includes the development of a mobile data acquisition device and a smartphone application to visualize the data collected from the stent. Thus, it is necessary to have a device that works as an NFC receiver, schematically represented in Figure 6. In an embodiment, the adopted approach involved the development of a mobile NFC reader, powered by a rechargeable battery **23**, capable of communicating via Bluetooth with the user's smartphone, presenting the data obtained through an app. The advantage of this option is that it allows to extend the reading range when compared to the NFC system integrated in the mobile phone.

On the present embodiment, the NFC Initiator/HF Reader **19** is a highly integrated IC, including the analog front end (AFE) and a highly integrated data framing system for ISO 18092 (NFCIP-1) initiator, ISO 18092 (NFCIP-1) active target, ISO 14443A and B reader (including high bit rates), ISO 15693 reader and FeliCa^{™} reader. It is intended to directly drive external antennas, and to detect transponder modulation superimposed on the 13.56 MHz carrier signal. A 4-wire Serial Peripheral Interface (SPI) is used for communication between the external microcontroller and the IC.

A microcontroller unit **20** manages the communication between the reader and the Bluetooth. It is connected with the IC reader through a SPI digital interface and with the Bluetooth by universal asynchronous receiver transmitter (UART) interface. By its turn, Bluetooth **21** transmits the data collected from de pressure sensor to the user's smartphone. As previously mentioned, it is connected to the microcontroller through an UART interface.

A USB connector **22** is used to charge, by cable, a rechargeable battery **23** that powers the wireless reader device. A charge management circuit **24**, a battery charge management system, integrates the most common functions for wearable and portable devices, namely a charger, a regulated output voltage rail for system power, and ADC for battery and system monitoring. It integrates advanced power path management and control that allows the device to provide power to the system while charging the battery.

The NFC reader antenna **25** of the external reader device is a loop antenna fabricated on a flexible substrate with 15 cm diameter and has an inductance of approximately 1 µH. The NFC antenna resonance frequency is adjusted with external tuning capacitors.

On second embodiment, a method of wireless energy transfer was considered to supply the stent. A wireless transmitter can induce energy to supply the electronic circuit, through an antenna integrated in the stent and a resonant circuit. Thus, the supply circuit has a wireless interface with the exterior, based on energy transfer by induction. This modular wireless energy interface supplies the acquisition system without using an energy harvesting NFC transceiver. It allows the use of a different wireless communication technology (such as Bluetooth, Wi-Fi, radiofrequency, ZigBee, etc.) and, therefore, improve the communication range of the implanted device. In this solution, the antenna used to transmit the acquired data is independent from the antenna used on the energy harvesting circuit.

To transmit data using Bluetooth technology, the DA14531 ultra-low power system-on-a-chip (SoC) integrating a 2.4 GHz transceiver from Dialog Semiconductor was selected. It can be used as a standalone application processor or as a data pump in hosted systems and is compatible with Bluetooth V5.1, ETSI EN 300 328 and EN 300 440 Class 2 (Europe), with a typical range of up to 10 meters. The antenna is a commercially available 2.4GHz chip antenna from Johanson Technology with 0.37 mm thickness. With this solution, the data is directly transmitted to the patient's smartphone.

Miniaturization of the circuit is achieved by the development of thin and flexible PCBs **26**, assembling the micro components on its surface. This flexible PCB **26** is fixed on the stent **1** (Figure 7) using a biocompatible epoxy and a thin heat shrink tubing, covering the entire system except for the pressure sensor.

After connecting the components and fixing the device to the stent, the circuit is encapsulated with a biocompatible surface coating material that allows the proper protection of the sensors without interfering with the measurement of physiological parameters (Figure 8).

Respecting the requirements mentioned above, the correct functioning of the system is promoted, as well as the user's comfort, also reducing the possibility of acute reactions to the implantation of the device.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof.

The above described embodiments are combinable.

The following claims further set out particular embodiments of the disclosure.

## Claims

1. An implantable biliary or pancreatic stent (1) for implanting in the gastrointestinal tract, comprising a first end (2) for placing in the bile duct or the pancreatic duct and a second end (3) for placing in the duodenum, the first end comprising a pressure sensor (4) arranged to measure bile duct or pancreatic duct pressure, respectively, and the second end comprising a pressure sensor (6) arranged to measure duodenal pressure, wherein the sensors are configured to detect a differential pressure between the bile duct (9) or the pancreatic duct (12), and the duodenum (10);
the implantable biliary or pancreatic stent (1) further comprising an electronic data processor arranged to calculate the differential pressure between the bile or pancreatic duct sensor (4), and the duodenum sensor (6);
wherein each pressure sensor (4, 6) comprises an electronic circuit with electronic components and a substrate for receiving the electronic circuit and electronic components, wherein said substrate is a flexible membrane;
wherein the flexible membrane is a flexible tube that is part of a thin tube that forms the stent, or the flexible membrane is a sleeve surrounding the stent.

2. The implantable biliary or pancreatic stent (1) according to the previous claim further comprising an electronic data processor arranged to detect and calculate pressure during bile discharge or pancreatic juice secretion.

3. The implantable biliary or pancreatic stent (1) according to any of the previous claims wherein the first end pressure sensor is configured to detect a relative pressure of 0 up to 200 cmH₂O (19.6 kPa) and wherein the second end pressure sensor to be placed in the duodenum (10) is configured to detect a relative pressure of 0 up to 100 cmH₂O (9.8 kPa).

4. The implantable biliary or pancreatic stent (1) according to any of the previous claims wherein the sensors are configured to detect a differential pressure between the bile duct (9) or the pancreatic duct (12), and duodenum (10) up to 200 cmH₂O (19.6 kPa).

5. The implantable biliary or pancreatic stent (1) according to any of the previous claims wherein the two sensors are electrically independent.

6. The implantable biliary or pancreatic stent (1) according to any of the previous claims wherein each pressure sensor comprises a flexible PCB having soldered electronic components.

7. The implantable biliary or pancreatic stent (1) according to any of the previous claims wherein one or more of said sensors (4, 6) comprises an antenna for receiving power wirelessly, preferably wherein the antenna is a near-field communication, NFC, antenna.

8. The implantable biliary or pancreatic stent (1) according to any of the previous claims wherein one or more of said sensors (4, 6) comprises an antenna for transmitting data wirelessly, preferably wherein the antenna is a near-field communication, NFC, antenna.

9. The implantable biliary or pancreatic stent (1) according to any of the previous claims further comprising a transmitter with an antenna that comprises an operation frequency of 6-60 MHz, in particular 13.56 MHz.

10. The implantable biliary or pancreatic stent (1) according to any of the previous claims comprising a liquid-tight encapsulation of said pressure sensors (4, 6).

11. The implantable biliary or pancreatic stent (1) according to any of the previous claims having a diameter inferior to 3 mm, preferably 2.5-2 mm.

12. The implantable biliary or pancreatic stent (1) according to any of the previous claims wherein the plurality of pressure sensors is selected from capacitive sensor, piezoresistive sensor, or combinations thereof.

13. The implantable biliary or pancreatic stent (1) according to any of the previous claims further comprising a pH sensor, a temperature sensor, a flow sensor, a volume sensor, or combinations thereof.

14. A kit comprising the implantable biliary or pancreatic stent (1) according to any of the previous claims and an external reader comprising an antenna and an electronic circuit for communicating wirelessly with said stent.

15. A manufacture method of an implantable biliary or pancreatic stent (1) according to any of the previous claims, comprising the steps of:
providing a biliary or pancreatic stent (1) for implanting in the gastrointestinal tract, comprising a first end (2) for placing in the bile duct (9) or the pancreatic duct (12) and a second end (3) for placing in the duodenum (10);
providing the first end with a pressure sensor (4) arranged to measure bile duct or pancreatic duct pressure, respectively, and providing the second end with a pressure sensor (6) arranged to measure duodenal pressure, wherein the sensors are configured to detect a differential pressure between the bile duct (9) or the pancreatic duct (12), and the duodenum (10);
providing an electronic data processor arranged to calculate the differential pressure between the bile or pancreatic duct sensor (4), and the duodenum sensor (6);
wherein each pressure sensor (4, 6) comprises an electronic circuit with electronic components and a substrate for receiving the electronic circuit and electronic components, wherein said substrate is a flexible membrane;
wherein the flexible membrane is a flexible tube that is part of a thin tube that forms the stent, or the flexible membrane is a sleeve surrounding the stent.

## Patentansprüche

1. Ein implantierbarer Gallen- oder Pankreasstent (1) für die Implantation in den Gastrointestinaltrakt, umfassend ein erstes Ende (2) zum Einsetzen in den Gallengang oder den Bauchspeicheldrüsengang und ein zweites Ende (3) zum Einsetzen in den Zwölffingerdarm, wobei das erste Ende einen Drucksensor (4) umfasst, der so angeordnet ist, dass er den Druck im Gallengang bzw. im Bauchspeicheldrüsengang misst, und das zweite Ende einen Drucksensor (6) umfasst, der so angeordnet ist, dass er den Druck im Zwölffingerdarm misst, wobei die Sensoren so konfiguriert sind, dass sie einen Differenzdruck zwischen dem Gallengang (9) oder dem Bauchspeicheldrüsengang (12) und dem Zwölffingerdarm (10) erfassen;
der implantierbare Gallen- oder Pankreasstent (1), ferner einen elektronischen Datenprozessor umfasst, der so beschaffen ist, dass er den Differenzdruck zwischen dem Sensor des Gallen- oder Bauchspeicheldrüsengangs (4) und dem Sensor des Zwölffingerdarms (6) berechnet;
wobei jeder Drucksensor (4, 6) eine elektronische Schaltung mit elektronischen Komponenten und ein Substrat zur Aufnahme der elektronischen Schaltung und der elektronischen Komponenten umfasst, wobei das genannte Substrat eine flexible Membran ist;
wobei die flexible Membran ein flexibles Rohr ist, das Teil eines dünnen Rohrs ist, das den Stent bildet, oder die flexible Membran eine Hülle ist, die den Stent umschließt.

2. Der implantierbare Gallen- oder Pankreasstent (1) nach dem vorangehenden Anspruch, ferner umfassend einen elektronischen Datenprozessor, der so angeordnet ist, dass er den Druck während des Austritts von Galle oder Pankreassaft erfasst und berechnet.

3. Der implantierbare Gallen- oder Pankreasstent (1) nach einem der vorangehenden Ansprüche, wobei der erste Enddrucksensor so konfiguriert ist, dass er einen relativen Druck von 0 bis zu 200 cmH₂O (19,6 kPa) erfasst, und wobei der zweite, im Zwölffingerdarm (10) zu platzierende Enddrucksensor so konfiguriert ist, dass er einen relativen Druck von 0 bis zu 100 cmH₂O (9,8 kPa) erfasst.

4. Der implantierbare Gallen- oder Pankreasstent (1) nach einem der vorangehenden Ansprüche, wobei die Sensoren so konfiguriert sind, dass sie einen Differenzdruck zwischen dem Gallen- (9) oder dem Bauchspeicheldrüsengang (12) und dem Zwölffingerdarm (10) von bis zu 200 cmH₂O (19,6 kPa) erfassen.

5. Der implantierbare Gallen- oder Pankreasstent (1) nach einem der vorangehenden Ansprüche, wobei die beiden Sensoren elektrisch unabhängig sind.

6. Der implantierbare Gallen- oder Pankreasstent (1) nach einem der vorangehenden Ansprüche, wobei jeder Drucksensor eine flexible Leiterplatte PCB mit verlöteten elektronischen Komponenten umfasst.

7. Der implantierbare Gallen- oder Pankreasstent (1) nach einem der vorangehenden Ansprüche, wobei einer oder mehrere der genannten Sensoren (4, 6) eine Antenne zum drahtlosen Energieempfang umfasst, wobei die Antenne bevorzugt eine Antenne für die Nahfeldkommunikation (NFC) ist.

8. Der implantierbare Gallen- oder Pankreasstent (1) nach einem der vorangehenden Ansprüche, wobei einer oder mehrere der genannten Sensoren (4, 6) eine Antenne zur drahtlosen Datenübertragung umfasst, wobei die Antenne bevorzugt eine Antenne für die Nahfeldkommunikation (NFC) ist.

9. Der implantierbare Gallen- oder Pankreasstent (1) nach einem der vorangehenden Ansprüche, ferner umfassend einen Sender mit einer Antenne, die eine Betriebsfrequenz von 6-60 MHz, insbesondere 13,56 MHz, aufweist.

10. Der implantierbare Gallen- oder Pankreasstent (1) nach einem der vorangehenden Ansprüche, der eine flüssigkeitsdichte Verkapselung der genannten Drucksensoren (4, 6) umfasst.

11. Der implantierbare Gallen- oder Pankreasstent (1) nach einem der vorangehenden Ansprüche, der einen Durchmesser von weniger als 3 mm, bevorzugt 2,5-2 mm aufweist.

12. Der implantierbare Gallen- oder Pankreasstent (1) nach einem der vorangehenden Ansprüche, wobei die Mehrzahl der Drucksensoren aus kapazitiven Sensoren, piezoresistiven Sensoren oder Kombinationen davon ausgewählt wird.

13. Der implantierbare Gallen- oder Pankreasstent (1) nach einem der vorangehenden Ansprüche, ferner umfassend einen pH-Sensor, einen Temperatursensor, einen Durchflusssensor, einen Volumensensor oder Kombinationen davon.

14. Ein Kit, umfassend den implantierbaren Gallen- oder Pankreasstent (1) nach einem der vorangehenden Ansprüche und ein externes Lesegerät, umfassend eine Antenne und eine elektronische Schaltung zur drahtlosen Kommunikation mit dem genannten Stent umfasst.

15. Ein Verfahren zur Herstellung eines implantierbaren Gallengangs- oder Pankreasstents (1) nach einem der vorangehenden Ansprüche, die folgenden Schritte umfassend:
Bereitstellen eines Gallen- oder Pankreasstents (1) für die Implantation in den Gastrointestinaltrakt, umfassend ein erstes Ende (2) zum Einsetzen in den Gallengang (9) oder den Bauchspeicheldrüsengang (12) und ein zweites Ende (3) zum Einsetzen in den Zwölffingerdarm (10);
Versehen des ersten Endes mit einem Drucksensor (4), der so angeordnet ist, dass er den Druck im Gallengang bzw. im Bauchspeicheldrüsengang misst, und Versehen des zweiten Endes mit einem Drucksensor (6), der so angeordnet ist, dass er den Druck im Zwölffingerdarm misst, wobei die Sensoren so konfiguriert sind, dass sie einen Differenzdruck zwischen dem Gallengang (9) oder dem Bauchspeicheldrüsengang (12) und dem Zwölffingerdarm (10) erfassen;
Bereitstellen eines elektronischen Datenprozessors, der so beschaffen ist, dass er den Differenzdruck zwischen dem Sensor des Gallen- oder Bauchspeicheldrüsengangs (4) und dem Sensor des Zwölffingerdarms (6) berechnet;
wobei jeder Drucksensor (4, 6) eine elektronische Schaltung mit elektronischen Komponenten und ein Substrat zur Aufnahme der elektronischen Schaltung und der elektronischen Komponenten umfasst, wobei das genannte Substrat eine flexible Membran ist;
wobei die flexible Membran ein flexibles Rohr ist, das Teil eines dünnen Rohrs ist, das den Stent bildet, oder die flexible Membran eine Hülle ist, die den Stent umschließt.

## Revendications

1. Une endoprothèse biliaire ou pancréatique implantable (1) destinée à être implantée dans le tractus gastro-intestinal, comprenant une première extrémité (2) destinée à être placée dans les canaux biliaires ou dans le canal pancréatique et une seconde extrémité (3) destinée à être placée dans le duodénum, la première extrémité comprenant un capteur de pression (4) disposé pour mesurer la pression des canaux biliaires ou du canal pancréatique, respectivement, et la seconde extrémité comprenant un capteur de pression (6) disposé pour mesurer la pression duodénale, dans laquelle les capteurs sont configurés pour détecter une pression différentielle entre les canaux biliaires (9) ou le canal pancréatique (12), et le duodénum (10) ;
l'endoprothèse biliaire ou pancréatique implantable (1) comprenant également un traiteur de données électronique disposé pour calculer la pression différentielle entre le capteur des canaux biliaires ou pancréatique (4), et le capteur du duodénum (6) ;
dans laquelle chaque capteur de pression (4, 6) comprend un circuit électronique avec des composants électroniques et un substrat pour recevoir le circuit électronique et les composants électroniques, dans laquelle ledit substrat est une membrane flexible ;
dans laquelle la membrane flexible est un tube flexible qui fait partie d'un tube fin qui forme l'endoprothèse, ou la membrane flexible est un manchon entourant l'endoprothèse.

2. L'endoprothèse biliaire ou pancréatique implantable (1) selon la revendication précédente comprenant également un traiteur de données électronique disposé pour détecter et calculer la pression durant l'évacuation de la bile ou la sécrétion du suc pancréatique.

3. L'endoprothèse biliaire ou pancréatique implantable (1) selon l'une quelconque des revendications précédentes dans laquelle le capteur de pression de la première extrémité est configuré pour détecter une pression relative de 0 jusqu'à 200 cmH₂O (19.6 kPa) et dans laquelle le capteur de pression de la seconde extrémité devant être placé dans le duodénum (10) est configuré pour détecter une pression relative de 0 jusqu'à 100 cmH₂O (9.8 kPa).

4. L'endoprothèse biliaire ou pancréatique implantable (1) selon l'une quelconque des revendications précédentes dans laquelle les capteurs sont configurés pour détecter une pression différentielle entre les canaux biliaires (9) ou le canal pancréatique (12), et le duodénum (10) jusqu'à 200 cmH₂O (19.6 kPa).

5. L'endoprothèse biliaire ou pancréatique implantable (1) selon l'une quelconque des revendications précédentes dans laquelle les deux capteurs sont électriquement indépendants.

6. L'endoprothèse biliaire ou pancréatique implantable (1) selon l'une quelconque des revendications précédentes dans laquelle chaque capteur de pression comprend une PCB flexible ayant des composants électroniques soudés.

7. L'endoprothèse biliaire ou pancréatique implantable (1) selon l'une quelconque des revendications précédentes dans laquelle un ou plusieurs desdits capteurs (4, 6) comprennent une antenne pour recevoir de l'énergie sans fils, préférablement dans laquelle l'antenne est une antenne de communication en champ propre, NFC.

8. L'endoprothèse biliaire ou pancréatique implantable (1) selon l'une quelconque des revendications précédentes dans laquelle un ou plusieurs desdits capteurs (4, 6) comprennent une antenne pour transmettre des données sans fils, préférablement dans laquelle l'antenne est une antenne de communication en champ propre, NFC.

9. L'endoprothèse biliaire ou pancréatique implantable (1) selon l'une quelconque des revendications précédentes comprenant également un transmetteur avec une antenne qui comprend une fréquence de fonctionnement de 6-60 Mhz, en particulier 13.56 MHz.

10. L'endoprothèse biliaire ou pancréatique implantable (1) selon l'une quelconque des revendications précédentes comprenant une encapsulation étanche desdits capteurs de pression (4, 6).

11. L'endoprothèse biliaire ou pancréatique implantable (1) selon l'une quelconque des revendications précédentes ayant un diamètre inférieur à 3mm, préférablement 2,5-2mm.

12. L'endoprothèse biliaire ou pancréatique implantable (1) selon l'une quelconque des revendications précédentes dans laquelle la pluralité de capteurs de pression est choisie parmi un capteur capacitatif, un capteur piézorésistif, ou des combinaisons de ceux-ci.

13. L'endoprothèse biliaire ou pancréatique implantable (1) selon l'une quelconque des revendications précédentes comprenant également un capteur de pH, un capteur de température, un capteur de débit, un capteur de volume, ou des combinaisons de ceux-ci.

14. Un kit comprenant l'endoprothèse biliaire ou pancréatique implantable (1) selon l'une quelconque des revendications précédentes et un lecteur externe comprenant une antenne et un circuit électronique pour communiquer sans fils avec ladite endoprothèse.

15. Un procédé de fabrication d'une endoprothèse biliaire ou pancréatique implantable (1) selon l'une quelconque des revendications précédentes, comprenant les étapes de :
fournir une endoprothèse biliaire ou pancréatique (1) destinée à être implantée dans le tractus gastro-intestinal, comprenant une première extrémité (2) destinée à être placée dans les canaux biliaires (9) ou dans le canal pancréatique (12) et une seconde extrémité (3) destinée à être placée dans le duodénum (10) ;
pourvoir la première extrémité d'un capteur de pression (4) disposé pour mesurer la pression des canaux biliaires ou du canal pancréatique, respectivement, et
pourvoir la seconde extrémité d'un capteur de pression (6) disposé pour mesurer la pression duodénale, dans laquelle les capteurs sont configurés pour détecter une pression différentielle entre les canaux biliaires (9) ou le canal pancréatique (12), et le duodénum (10) ;
fournir un traiteur de données électronique disposé pour calculer la pression différentielle entre le capteur des canaux biliaires ou pancréatique (4), et le capteur du duodénum (6) ;
dans laquelle chaque capteur de pression (4, 6) comprend un circuit électronique avec des composants électroniques et un substrat pour recevoir le circuit électronique et des composants électroniques, dans laquelle ledit substrat est une membrane flexible ;
dans laquelle la membrane flexible est un tube flexible qui fait partie d'un tube fin qui forme l'endoprothèse, ou la membrane flexible est un manchon entourant l'endoprothèse.
